# EUROPEAN PATENT APPLICATION

(11) **EP 1 639 965 A1**
(43) Date of publication of application: **29.03.2006**
(21) Application number: 05255809.5
(22) Date of filing: 21.09.2005
(51) Int. Cl.: A61F 2/40

(54) **Extended articulation prosthesis adaptor**

(30) Priority: 27.09.2004 US 951023
(71) Applicant: DePuy Products, Inc., Warsaw, IN 46581 (US)
(72) Inventor: Ondrla, Jeffrey M., N. Leesburg, IN 46538 (US); Shoup, Jared R., Cordova, TN 38016 (US)
(74) Representative: Belcher, Simon James

(57) **Abstract**

A shoulder arthroplasty kit (100) for shoulder arthroplasty includes a stem (102) for insertion into the humerus and a first member (104). The first member has a surface (106) having a convex periphery (108) adapted for articulation with the natural glenoid fossa (8). The convex periphery includes a first articulating surface (110) defining a generally circular outer periphery of the first articulating surface and a second articulating surface (112) extending from a portion of the circular outer periphery of the first articulating surface. The first member is removably cooperable with the stem. The kit also includes a second member (114) including a portion having a concave periphery (116). The second member is removably cooperable with the stem. The kit further includes a third member (118) for insertion into the natural glenoid fossa. The third member includes a portion having a convex periphery (120). The third member is adapted for articulation with the second member.

## Description

The invention relates to implantable articles for implanting such articles. More particularly, the invention relates to a bone prosthesis.

There are known to exist many designs for and methods of implanting implantable articles, such as bone prostheses. Such bone prostheses include components of artificial joints, such as elbows, hips, knees and shoulders.

Early designs of implantable articles relied upon the use of cements to anchor the implant. However, the current trend is to use cements to a lesser extent because of their tendency to lose adhesive properties over time and the possibility that cement contributes to wear debris within a joint.

Recently, implantable bone prostheses have been designed such that they encourage the growth of hard bone tissue around the implant. Such implants are often implanted without cement and the bone grows around surface irregularities, for example, porous structures on the implant.

One such implantable prosthesis is a shoulder prosthesis. During the lifetime of a patient it may be necessary to perform a total shoulder replacement procedure on a patient as a result of, for example, disease or trauma, for example, disease from osteoarthritis or rheumatoid arthritis. Currently, most implantable shoulder prostheses are total shoulder prostheses. In a total shoulder replacement procedure, a humeral component having a head portion is utilized to replace the natural head portion of the upper arm bone or humerus. The humeral component typically has an elongated intramedullary stem, which is utilized to secure the humeral component to the patient's humerus. In such a total shoulder replacement procedure, the natural glenoid surface of the scapula may be resurfaced or otherwise replaced with a glenoid component that provides a bearing surface for the head portion of the humeral component.

With the average age of patients requiring shoulder athroplasty decreasing, device manufacturers are developing bone sparing implants for the initial treatment of degenerative arthritis. Surface replacement prostheses are being developed to replace the articulating surface of the proximal humerus with a minimal bone resection and minimal disruption of the metaphysis and diaphysis. Current designs utilize a semi-spherical articular dome with a small stem for rotational stability. The under surface of the articular head is also semi-spherical and mates with the spherically machined humeral head.

The need for a shoulder replacement procedure may be created by the presence of one of a number of conditions. One such condition is the deterioration of the patient's rotator cuff. Specifically, an intact rotator cuff stabilizes the humeral head in the glenoid fossa of a scapula during abduction of the arm. While it is stabilized in such a manner abduction of the arm causes the humeral head to translate only a short distance in the superior direction (e.g. a few millimetres), whereby a space is maintained between the humeral head and the acromion. However, for patients with rotator cuff arthropathy, significantly greater humeral excursion is observed.

In particular, hyper-translation of the humeral head in the superior direction is observed in patients with massive rotator cuff deficiency, thereby resulting in articulation between the superior surface of the humeral head and both the inferior surface of the acromion and the acromioclavicular joint during abduction of the patient's arm. Such articulation between these components accelerates humeral articular destruction and the erosion of the acromion and acromioclavicular joint. Moreover, such bone-to-bone contact is extremely painful for the patient, thereby significantly limiting the patient's range of motion. In short, patients with massive rotator cuff tear and associated glenohumeral arthritis, as is seen in cuff tear arthropathy, may experience severe shoulder pain, as well as reduced function of the shoulder.

In order to treat patients suffering from cuff tear arthropathy, a number of prostheses and techniques utilizing existing prostheses have heretofore been designed. For example, surgeons heretofore utilized a relatively large humeral head prosthesis in an attempt to completely fill the shoulder joint space. It was believed that such use of a large prosthesis would increase the efficiency of the deltoid muscle, thereby improving motion of the shoulder. However, clinical experience has shown that such use of a large humeral head prosthesis (overstuffs) the shoulder joint thereby increasing soft tissue tension, reducing joint range of motion, and increasing shoulder pain. Moreover, such use of an oversized prosthetic head fails to resurface the area of the greater tubercle of the humerus, thereby allowing for bone-to-bone contact between the greater tubercle and the acromion during abduction of the patient's arm.

A number of humeral head bipolar prostheses have also been utilized in an attempt to address the problems associated with cuff tear arthropathy. It was believed that the relatively unstrained motion of the bipolar head would improve shoulder motion. However, heretofore designed bipolar prosthetic heads include relatively large offsets, thereby overstuffing the shoulder joint in a similar manner as described above. Moreover, scar tissue may form around the bipolar head thereby (freezing) the dual articulating motion of the prosthesis that has been known to create a large hemi arthroplasty that likewise overstuffs the shoulder joint. In addition, such bipolar prosthetic heads do not cover the articulating surface between the greater tubercle and the acromion, thereby creating painful bone-to-bone contact between them.

Yet further, a number of techniques have heretofore been designed in which the relatively rough surface of the greater tubercle is resurfaced with an osteotome or high speed burr. Although this approach results in a smoother tubercle contact surface, relatively painful bone-to-bone articulating contact still occurs, thereby reducing the patient's range of motion.

Apparatus for treating cuff tear arthroplasty utilizing a total shoulder replacement prosthesis is disclosed in US-A-2002/0099445. The apparatus includes a prosthesis which has an artificial head as well as a stem that extends into a reamed medullary canal. Such a prosthesis is limited to use with a total shoulder prosthesis and is not suitable for use with bone sparing implants for the initial treatment of the degenerative arthritis.

One problem faced by both conventional and modular prostheses is the deterioration of the shoulder joint that can accompany a shoulder athroplasty. For instance, a patient who has under gone shoulder arthoplasty may experience a loss of soft tissue strength, which could eventually lead to total loss of the key constraints that contain the joint. This loss of soft tissue and soft tissue strength can allow unnatural joint loads to be produced, which can compromise the function of the prosthetic joint, and can lead to pain.

One solution for this problem is the revision of the shoulder prosthesis. This revision can entail the substitution of different articulating components, or differently sizes components. One treatment, the shoulder prosthesis has changed to a reverse type prosthesis. A typical prosthetic shoulder replicates the anatomy of the joint. Specifically, the humeral component provides a convex articulate surface, much like the natural end of a humerus. This convex surface mates with the concave glenoid component. A reverse type prosthesis essentially reverses the arrangement of the articulating surfaces. Specifically, the glenoid component includes a convex or partially a concave spherical component while the humeral head includes a concave spherical component. One consideration involved in the use of a reverse prosthesis is that the concave articulating surface that is now part of the humeral component, may actually protrude in the metaphyseal region of the humerus. This modified geometry can require modification of the metaphyseal portion of the bone as well as the prostheses.

In order to address these needs, prior systems have required total revision of the joint. A total revision entails removal of the entire humerus including the stem that is fixed in the diphyseal of the implant. Of course, this surgery procedure is very difficult and invasive, and can put the patient and the shoulder joint at risk.

Most patients with massive rotator cuff tears have proximal migration of the humerus, limited range of motion of the joint, and are in pain. The current methods of treatments for these patients are a standard hemiathroplasty, a total shoulder arthoplasty with a cuff tear athroplasty head, or a reversed total shoulder athroplasty (RTSA) with a reversed total shoulder implant such as that sold by DePuy Orthopaedics, Warsaw, IN under the trade mark DELTA.

There are no options for the surgeon to conservatively treat these patients that allow for the conversion of hemiathroplasty with a cuff tear athroplasty head to a reverse total shoulder athroplasty.

What is needed, therefore, is apparatus for performing bone sparing arthroplasty shoulder replacement surgery utilizing bone sparing implants for the initial treatment of degenerative arthritis, which will be useful in the treatment of cuff tear arthroplasty, which overcomes one or more of the aforementioned drawbacks. What is particularly needed is a method and apparatus for performing a bone sparing implant shoulder procedure that eliminates painful articulation between the great tubercle of the humerus and the acromion.

According to the present invention, an alternate solution to the basic total shoulder replacement is provided for a patient in which an irreparable rotator cuff tear or cuff tear athroplasty of the shoulder is needed. The present invention allows a surgeon to convert between a cuff tear athroplasty head on a reverse stem to the reversed geometry designed using the reversed or Delta stem and a cuff tear arthopy (CTA) extended humeral head. In an aspect of the present invention, an adaptor is provided between the locking interface of the reverse humeral stem component and the locking taper of the cuff tear arthropathy humeral head which allows for the use of an extended cuff tear arthropathy head to be used on the reverse Delta epiphyseal component.

In one aspect, the invention provides a shoulder arthroplasty kit for providing for shoulder arthroplasty, comprising:
a stem for insertion into the humerus;
a first member including a surface having a convex periphery adapted for articulation with the natural glenoid fossia, the convex periphery including a first articulating surface defining a generally circular outer periphery thereof and a second articulating surface extending from a portion of the circular outer periphery of the first articulating surface, said first member being removably cooperable with said stem;
a second member including a portion having a concave periphery, said second member being removably cooperable with said stem; and
a third member for insertion into the natural glenoid fossia including a portion having a convex periphery, said third member being adapted for articulation with said second member.

In another aspect, the invention provides a shoulder prosthesis stem kit comprising:
a stem for insertion into the humerus;
a first member including a surface having a convex periphery adapted for articulation with the natural glenoid fossia, the convex periphery including a first articulating surface defining a generally circular outer periphery thereof and a second articulating surface extending from a portion of the circular outer periphery of the first articulating surface, said first member being removably cooperable with said stem; and
a second member including a portion having a concave periphery, said second member being removably cooperable with said stem.

In a further aspect, the invention provides a shoulder prosthesis stem comprising:
a stem for insertion into the humerus;
an adapter removably connected to said stem; and
a first member including a surface having a convex periphery adapted for articulation with the natural glenoid fossia, the convex periphery including a first articulating surface defining a generally circular outer periphery thereof and a second articulating surface extending from a portion of the circular outer periphery of the first articulating surface, said first member being removably connected to said adapter.

Apparatus provided by the invention can be used in a method of treatment for shoulder cuff tear arthropathy comprising:
providing a shoulder prosthesis kit including a stem for insertion into the humerus, a first member including a surface having a convex periphery adapted for articulation with the natural glenoid fossia, the convex periphery including a first articulating surface defining a generally circular outer periphery thereof and a second articulating surface extending from a portion of the circular outer periphery of the first articulating surface, said first member being removably cooperable with said stem, a second member including a portion having a concave periphery, said second member being removably cooperable with said stem, and a third member for insertion into the natural glenoid fossia including a portion having a convex periphery, said third member being adapted for articulation with said second member.
cutting an incision in the patient;
preparing the humeral cavity;
assembling the first member to the stem;
inserting the first member and stem into the humeral cavity;
sealing the incision;
monitoring the condition of the patient;
determining when one of the first member and the natural glenoid fossia are in need of replacement;
removing the first member from the stem;
placing the second member on the stem; and
placing the third member on the natural glenoid fossia.

According to one embodiment of the present invention, there is provided a shoulder arthroplasty kit for shoulder arthroplasty. The kit includes a stem for insertion into the humerus and a first member. The first member has a surface having a convex periphery adapted for articulation with the natural glenoid fossa. The convex periphery includes a first articulating surface defining a generally circular outer periphery of the first articulating surface and a second articulating surface extending from a portion of the circular outer periphery of the first articulating surface. The first member is removably cooperable with said stem. The kit also includes a second member including a portion having a concave periphery. The second member is removably cooperable with the stem. The kit further includes a third member for insertion into the natural glenoid fossia. The third member includes a portion having a convex periphery. The third member is adapted for articulation with the second member.

According to another embodiment of the present invention there is provided a shoulder prosthesis stem kit including a stem for insertion into the humerus and a first member. The first member includes a surface having a convex periphery adapted for articulation with the natural glenoid fossia. The convex periphery includes a first articulating surface defining a generally circular outer periphery of the first articulating surface and a second articulating surface extending from a portion of the circular outer periphery of the first articulating surface. The first member is removably cooperable with the stem. The kit also includes a second member including a portion having a concave periphery. The second member is removably cooperable with said stem.

According to still another embodiment of the present invention there is provided a shoulder prosthesis stem including a stem for insertion into the humerus and an adapter removably connected to the stem. The shoulder prosthesis stem also includes a first member having a surface having a convex periphery adapted for articulation with the natural glenoid fossia. The convex periphery includes a first articulating surface defining a generally circular outer periphery of the first articulating surface and a second articulating surface extending from a portion of the circular outer periphery of the first articulating surface. The first member is removably connected to the adapter.

According to a further embodiment of the present invention, there is provided a method of treatment for shoulder cuff tear arthropathy. The method includes the step of providing a shoulder prosthesis kit including a stem for insertion into the humerus and a first member including a surface having a convex periphery adapted for articulation with the natural glenoid fossia. The convex periphery includes a first articulating surface defining a generally circular outer periphery thereof and a second articulating surface extending from a portion of the circular outer periphery of the first articulating surface. The first member is removably cooperable with the stem.

The kit also includes a second member including a portion having a concave periphery. The second member is removably cooperable with the stem. The kit also includes a third member for insertion into the natural glenoid fossia including a portion having a convex periphery. The third member is adapted for articulation with said second member.

The method in which the apparatus of the invention might be used can include the steps of cutting an incision in the patient, preparing the humeral cavity, assembling the first member to the stem, inserting the first member and stem into the humeral cavity, and sealing the incision. The method can also include the steps of monitoring the condition of the patient, determining when one of the first member and the natural glenoid fossia are in need of replacement, removing the first member from the stem, placing the second member on the stem, and placing the third member on the natural glenoid fossia.

The technical advantages of the present invention include the ability to treat cuff deficient or cuff tear arthropathy of patients conservatively. For example, according to one aspect of the present invention, a shoulder athroplasty for providing shoulder athroplasty is provided. The kit includes a stem for insertion into the humerus and a first member including a surface having a convex periphery adapted for articulation with the globoid fossa. The kit further includes a second member having concave periphery and a third member for insertion into the natural glenoid fossa including a portion having a convex periphery. The kit further includes a fourth member for insertion into the glenoid fossa including a portion having a concave periphery. Fourth member is cooperative with the humerus. Fourth member is adapted for use with the third member as well as with the natural glenoid.

Thus, the present invention provides for the ability to treat cuff deficit or cuff tear arthropathy of the patient conservatively.

The technical advantages of the present invention also include the ability to use common extended humeral heads in both normal and reverse stem configurations. For example, according to another aspect of the present invention, a shoulder athroplasty kit is provided for shoulder athroplasty. The kit includes a stem for insertion into the humerus and a first member including a surface having convex periphery adapted for articulation with the glenoid fossa. The kit further includes a second member having a portion having a concave periphery. The second member is removably cooperable with the stem. The kit further includes a third member for insertion into the natural glenoid and includes a portion having a convex periphery.

The kit further includes an adaptor positional between the stem and either the first member or the second member for cooperation with the stem and the first member or the second member. The adaptor provides for the utilization of normal and reverse stem configurations. Thus, the present invention provides the ability to use the common extended humeral head in both normal and reverse stem configurations.

The technical advantages further include the ability to provide two different treatment methods while having a humeral stem remain in the patient. For example, according to another aspect of the present invention, a shoulder athroplasty kit is provided for shoulder athroplasty. The kit includes a stem for insertion into the humerus and a first member removably cooperable with the stem. The first member has a convex periphery for articulation with the glenoid fossa. The kit further includes the second member with a portion having a concave periphery. A second member is removably cooperable with the stem. The first member has the convex periphery that cooperates with the concave glenoid component while the second member includes a concave periphery which cooperates with a glenoid component having a convex periphery. Thus, the present invention provides for two different treatment methods with a common humeral stem remaining in the patient.

The technical advantages of the present invention further includes the ability to provide a series of anatomically different shoulder prostheses with a common humeral stem. For example, according to another aspect of the present invention, a shoulder athroplasty kit is provided with a stem for insertion into the humerus. The kit also includes first member including a surface having a convex periphery removably cooperable with the stem. The kit further includes a second member having a concave periphery and likewise being removably cooperable with the stem. The kit further includes an adaptor positional between the stem and the first member or the second member for cooperation with the stem and the first member or the second member. The kit may further include a second adaptor having at least one dimension different than the first adaptor. Thus, the present invention provides for anatomically different humeral prostheses with a common humeral stem. Each of the adaptors provide a different anatomical result while using a common head.

The technical advantages of the present invention further include the ability to provide components to create a greater number of potential options for the surgeon with fewer components. For example, according to yet another aspect of the present invention, a shoulder athroplasty kit is provided including a stem for insertion into the humerus as well as a first member with a convex periphery to cooperate with the stem. The kit further includes a second member having a concave periphery to likewise cooperate with the stem. The kit further includes a third component for insertion into the natural glenoid fossa adapted for articulation with the second member. The kit further includes a plurality of adaptors, of first members and of second members such that a variety of components can be selected. Thus, the present invention provides for a variety of components with a greater number of potential options with fewer components.

Embodiments of the invention will now be described by way of example with reference to the accompanying drawings, in which:
FIG. 1 is a plan view of a kit of prosthetic components, in accordance with an embodiment of the present invention including a reverse humeral prosthesis stem, an extended articulation head and a convex glenoid component;
FIG. 2 is a plan view of the reverse humeral prosthesis stem of the kit of FIG. 1 with a concave head;
FIG. 3 is an exploded perspective view of the reverse humeral prosthesis of the kit of FIG. 1 including the stem and the reverse humeral head of the kit of FIG. 1;
FIG. 4 is a plan view of the extended articulation head of the kit of FIG. 1;
FIG. 5 is a plan view of the convex glenoid component of the kit of FIG. 1;
FIG. 6 is a kit of prosthetic components in accordance with another embodiment of the present invention providing for components to treat a plurality of disease states;
FIG. 7 is a plan view of an adaptor of the kit of FIG. 6;
FIG. 8 is a plan view of the adaptor of FIG. 7 in position on the stem of the kit FIG. 6;
FIG. 9 is a plan view of a humeral prosthesis including the stem, the adaptor, and the extended articulation head of the kit of FIG. 6;
FIG. 10 is a plan view of a extended articulation conservative head of the kit of FIG. 6 in position on a humerus;
FIG. 11 is a plan view of the convex humeral head for use with the stem of the kit of FIG. 6;
FIG. 12 is a plan view of the metal-backed convex glenoid component of the kit of FIG. 6;
FIG. 13 is a plan view of the one-piece concave glenoid component of the kit of FIG. 6;
FIG. 14 is a plan view of the metal-backed concave glenoid component of the kit of FIG. 6;
FIG. 15 is a plan view of a shoulder joint with a diseased humerus representing a first disease state;
FIG. 16 is a plan view of a shoulder joint with a diseased humerus articulating upon the acromion representing a second disease state;
FIG. 17 is a plan view of shoulder joint with a diseased humerus and a diseased glenoid representing a third disease state;
FIG. 18 is a plan view of the conservative head of the kit of FIG. 6 is position or a humerus;
FIG. 19 is a plan view of humeral stem of FIG. 6 and the convex head of FIG. 6 assembled to each other and positioned is the stem of a humerus;
FIG. 20 is a plan view of the extended articulated conservative head of the kit of FIG. 6 assembled onto a humeral head;
FIG. 21 is a plan view of the extended articulation conservative head of the kit FIG. 6 assembled onto a humeral head and concave glenoid component assembled onto the glenoid cavity;
FIG. 22 is a plan view of a humeral prosthesis including the stem, the adaptor, and the extended articulation head of the kit of FIG. 6 in position on a humerus and in cooperation with a natural glenoid;
FIG. 23 is a plan view of a humeral prosthesis including the stem, the adaptor, and the extended articulation head of the kit of FIG. 6 in position on a humerus and in cooperation with a glenoid prosthesis of the kit of FIG. 6;
FIG. 24 is a plan view of a reverse humeral prosthesis including the stem, the adaptor, and the reverse humeral head of the kit of FIG. 6 in position on a humerus and in cooperation with the reverse glenoid prosthesis of the kit of FIG. 6;
FIG. 25 is an exploded plan view of a humeral prosthesis including the stem, the adaptor, and the extended articulation head of the kit of FIG. 6;
FIG. 26 is an exploded plan view of a reverse humeral prosthesis including the stem and the reverse humeral head of the kit of FIG. 6;
FIG. 27 is a partial plan view of a humeral prosthesis including a stem and an extended articulation head in accordance with another embodiment of the present invention;
FIG. 28 is a partial plan view of a reverse humeral prosthesis including the stem of FIG. 15 and an reverse humeral head in accordance with another embodiment of the present invention;
FIG. 29 is a partial plan view of a reverse humeral prosthesis including the stem of FIG. 15, an adaptor, and the reverse humeral head of FIG 1 in accordance with yet another embodiment of the present invention;
FIG. 30 is a plan view of a kit of prosthetic components, in accordance with yet another embodiment of the present invention including a standard humeral prosthesis stem, an adaptor, a reverse humeral head, an extended articulation head, and a standard articulation head; and
FIG. 31 is a flow chart for a method of performing shoulder arthroplasty in accordance to yet another embodiment of the present invention.

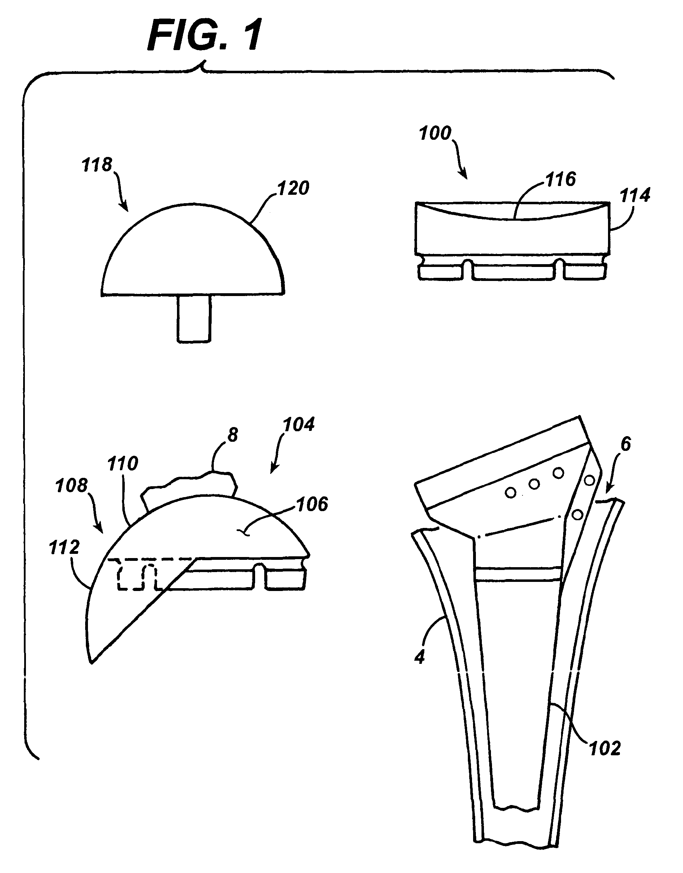

Referring to the drawings, FIG. 1 shows a shoulder athroplasty kit for providing shoulder athroplasty. The kit 100 includes a stem 102 for insertion into the humerus 4. The stem 102 may have any suitable size and shape to be adapted for cooperation with the humeral canal 6 of humerus 4. The stem 102 may be made of any suitable, durable material and may, for example, be made of a metal. If made of a metal, the stem 102 may be made of, for example, a cobalt chromium alloy, a stainless steel alloy, or a titanium alloy.

The kit 100 includes a first member 104. The first member 104 includes a surface 106 having a convex periphery. The surface 106 is adapted for articulation the glenoid fossa 8. The convex periphery 108 includes a first articulating surface 110 defining a generally an arcuate outer periphery thereof. The convex periphery 108 further includes a second articulating surface 112 extending from the first articulating surface 110. The first member 104 is removably cooperable with the stem 102.

The shoulder athroplasty kit 100 further includes a second member 114. A portion of the second member 114 has a concave periphery 116. The second member 114 is removably cooperable with the stem 102.

The shoulder athroplasty kit 100 also includes a third member 118 for insertion into the natural glenoid fossa including a portion having a convex periphery 120. The third member 118 is adapted for articulation with the second member 114.

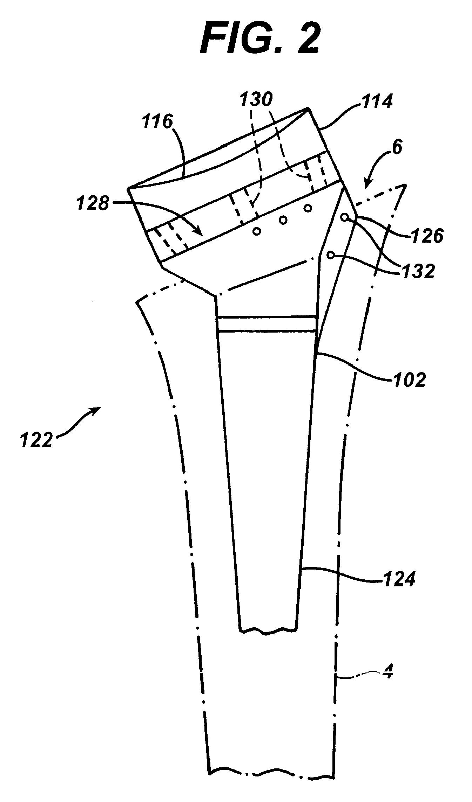

Referring now to FIG. 2, the second member 114 is shown installed into stem 102. The second member 114 and the stem 102 form concave humeral stem assembly 122.

The stem 102 may have any suitable shape and may as shown in FIG. 2 include a distal stem 124 which extends from a body 126. The distal stem 124 is preferably sized to canal 6 of the humerus. The distal stem 124 may be sized for cemented or cementless installation of the stem 102 into the canal 6.

The body 126 of the stem 102 may have any suitable shape and as shown in FIG. 2 may include a stem connecting feature 128 for connecting the second member 114 to the stem 102. Similarly, the second member 114 may include a head connecting feature 130 for connecting the second member 114 to the stem 102. The stem connecting feature 128 and the head connecting feature 130 may have any suitable shapes for cooperating with each other. For example and is shown in FIG. 2, the stem connecting feature 128 may be in the form of a tapered cavity while the head connecting feature 130 may be in the form of a tapered protrusion.

The body 126 of the stem 102 may, for example, and is shown in FIG. 2, include features 132 in the form of, for examples, holes or openings for receiving sutures to assist in the attachment of soft tissue.

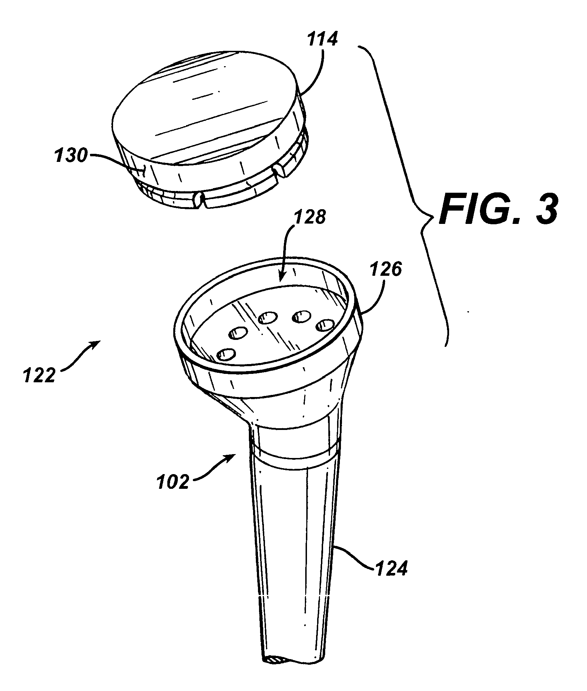

Referring now to FIG. 3, the concave humeral stem assembly 122 is shown in an exploded view. As shown in FIG. 3, the stem connecting feature 128 is shown in the form of the tapered cavity for receiving tapered periphery 130 of the second member 114.

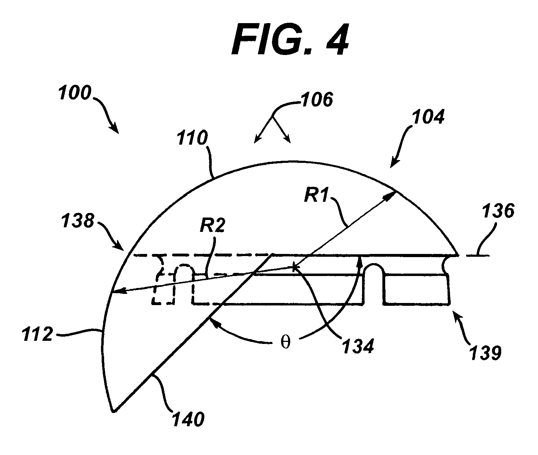

Referring now to FIG. 4, the first member 104 is shown in greater detail. The first member 104 includes the arcuate surface 106 which, as shown in FIG. 4, is defined by the first articulating surface 110 and the second articulating surface 112. The first articulating surface 110 may as shown in FIG.4, may be defined by a radius R 1 extending from origin 134. The second articulating surface, may similarly, be defined by a R2 by extending origin 134. The radius R1 and R2 may as shown in FIG. 4 be identical. The periphery of the first articulating surface may be defined by plane 136. As shown in FIG. 4, the plane 136 defines a boundary portion 138 positioned by the first articulating surface 110 and the second articulating surface 112. The boundary portion as shown in FIG. 4, is preferably generally smooth and continuous.

As shown in FIG. 4, the second articulating surface 112 further defines a second surface periphery 140 opposed to the first articulating surface 110. The second surface periphery 140 defines a second plane. The second plane 140 with the first plane 136 are non-consistent. As shown in FIG. 4, the first plane 136 and the second plane 140 define an included angle therebetween. As shown in FIG. 4, the included angle θ is greater than 90° or obtuse. As shown In FIG. 4, the angle θ may be for example, may be from 118 to 160°.

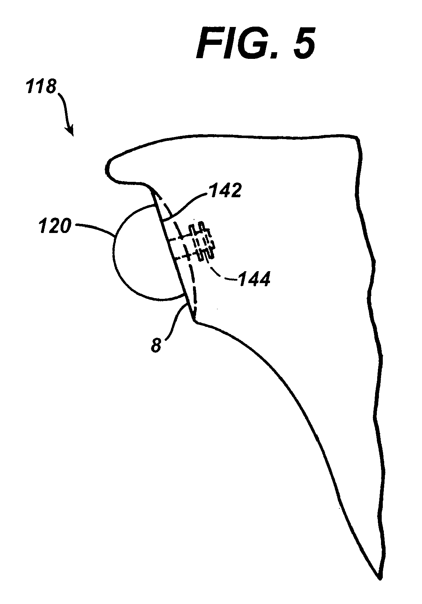

Referring now to FIG. 5, the third member 118 is shown in greater detail. The third member 118 includes a support surface 142 which sits against glenoid fossa 8. The third member 118, as shown in FIG. 5, include a support feature 144 for in the form of, for example, a protrusion. The third member 118 may be made of any suitable, durable material and may, for example, be made of a metal, a composite material, or a plastic. If made of a plastic, the third member may be made of, for example, ultra high molecular weight polyethylene.

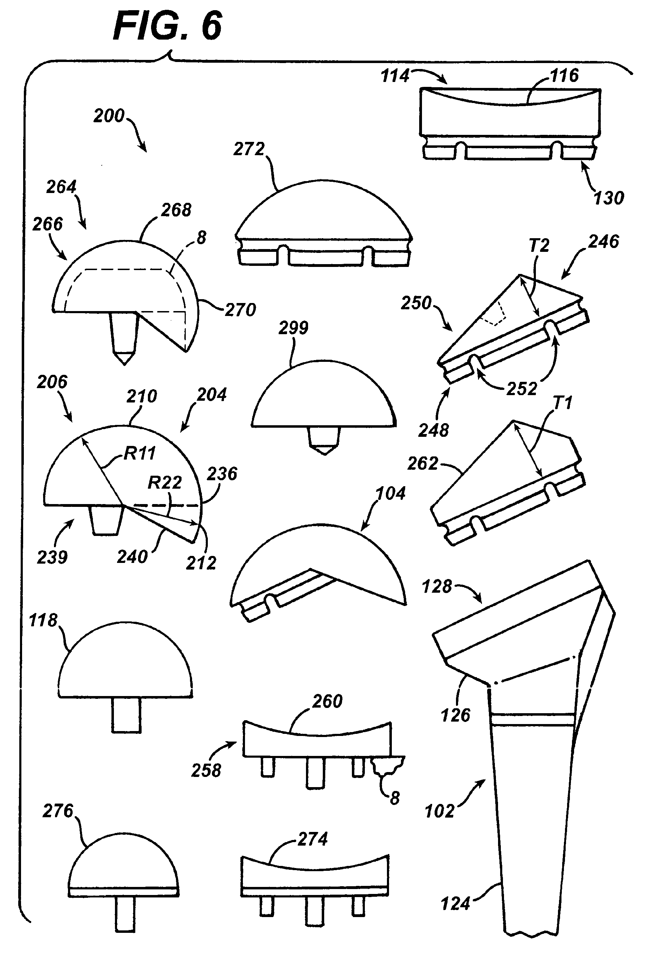

According to the present invention and referring now to FIG. 6, another embodiment of the present invention is shown as shoulder athroplasty kit 200. The kit 200 is utilized to assist in providing shoulder athroplasty. The shoulder athroplasty kit 200 includes the stem 102 of the kit 100 of FIGS. 1-5. The kit 200 further includes the second member 114 of the kit 100 of FIG. 1-5. The kit 200 also includes the first member 104 of the kit 100 of FIGS. 1-5 of the kit 100 of FIGS. 1-5.

Unlike the kit 100 of FIGS. 1-5 the kit 200 includes a first member 204 which is somewhat different than the first member 104 of the kit 100 of FIGS. 1-5. For example and as is shown in FIG. 6, the kit 200 includes a first member 204 including a surface 202 having a first articulating surface 210 defining outer periphery forming plane 236. The first member 204 further includes a second articulating surface 212 extending from the portion of the outer periphery of the first articulating surface 210. The periphery of the second articulating surface defines a second plane 240 which is non-coincident with the first plane 236.

The first articulating surface 210 may be defined by radius R11 while the second articulating surface 112 may be defined R22. In second member 204 unlike the first member 104 is not mateable with the stem 102. The first member 204 includes a connecting feature 239 which is different than the connecting feature 139 of the first member 104 of FIG. 4. The connecting feature 239 of the first member 204 may be in the form of a tapered protrusion extending from the first member 204.

As shown in FIG. 6, the shoulder athroplasty kit 200 further includes an adaptor 246 that may be positioned between the stem 102 and the first member 204. It should be appreciated that the adaptor 246 may be utilized to provide for a variety of overall lengths for the stem assembly and to provide for different orientations of the head or first member 204 with respect to stem 102. It should further be appreciated that the adaptor 246 provides for a first member 204 to be adaptable to the stem 102 regardless of the connecting mechanism 239 of the first member 204. Thus, the adaptor 246 may be utilized to provide for a first member 204 that may also be used with a stem of a totally different design.

The adaptor 246 includes a stem connecting feature 248 to connecting the adaptor 246 to the stem 202. The adaptor 246 may further include a head connecting feature 250 for connecting the adaptor 246 to the head or first member 204.

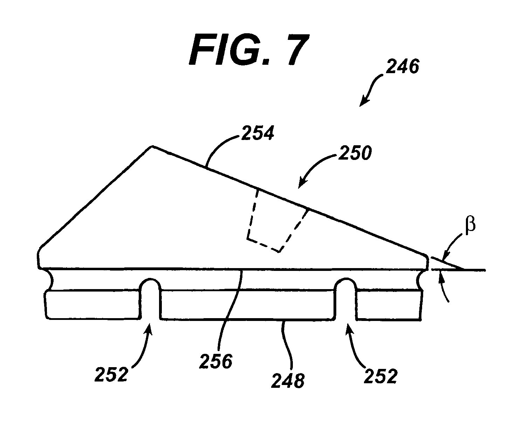

Referring now to FIG. 7, the adaptor 246 of the kit 200 is shown in greater detail. The adaptor 246 includes the stem connecting feature 248. The stem connecting feature 248 is constructed to secure the adaptor 246 to the stem 102. Thus, the stem connecting feature 248 is preferably similar to the stem connecting feature 130 of the second member 114 of the kit 100 of FIG. 1. For example and is shown in FIG. 7, the stem connecting feature 248 includes an external tapered lip. The lip 248 may include a plurality of spaced apart slots 252 to permit sufficient pliability to the stem connecting feature 248.

As shown in FIG. 7, the head connecting feature 250 may be positioned along first member connecting surface 254. It should be appreciated that in order to provide the first member 204 in the proper orientation with respect to the stem 102, the adaptor 246 may be configured such that the first member connecting surface 254 may be at an angle, for example, angle β with respect to the distal face 256 of the adaptor 246. The angle β may, for example, be 0 to 60° and, for example, may be approximately 15 to 45°.

The first member connecting feature 250 of the adaptor 246 is preferably configured to mate with the first member connecting feature 239 of the first member 204. For example and is shown in FIG. 7, the first member connecting feature 239 is in the form of a tapered cavity.

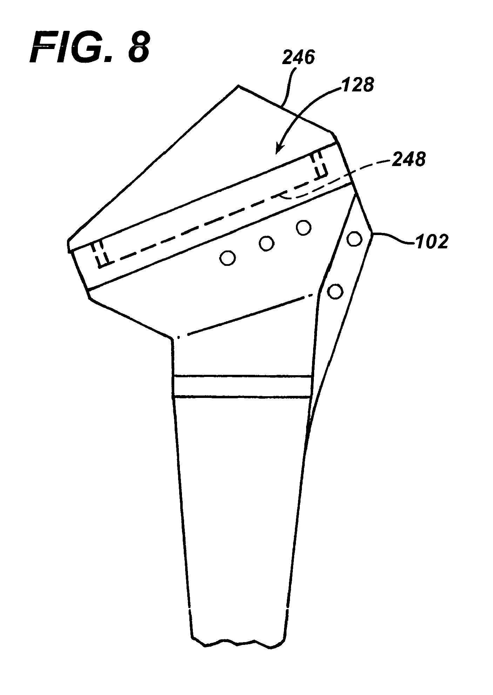

According to the present invention and referring now to FIG. 8, the adaptor 246 is shown in position on the stem 102. The stem connecting feature 248 is positioned inside the stem connecting feature 128 of the stem 102.

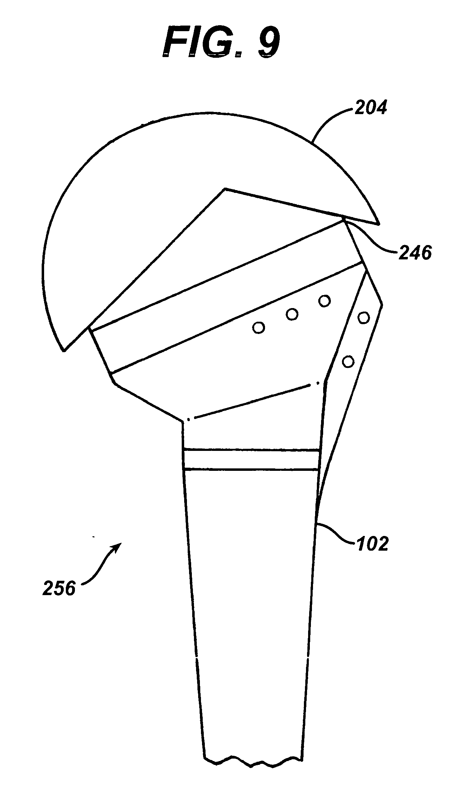

Referring now to FIG. 9, the first member 204 is shown in position on the adaptor 246 which is in position on the stem 102. The first member 204, the adaptor 246, and the stem 102 combine to form convex humeral stem assembly 256.

It should be appreciated that since the second member 114 and the stem 102 are components of the kit 200 of FIG. 6, the stem 102 and the second member 114 may be utilized with the kit 200 to form the concave humeral stem assembly 122 of FIG. 2.

Referring again to FIG. 6, the shoulder athroplasty kit 200 may further include a fourth component or concave glenoid component 258. The concave glenoid component 258 is utilized for insertion into the natural glenoid fossa 8. The concave glenoid component 258 includes a portion having a concave periphery 260. The fourth component is adapted for articulation with the first member 204.

The kit 200 may include, in addition to the first mentioned adaptor 246, a second adaptor 262 positionable between the stem 102 and the first member 204. The second adaptor 262 may be similar to the first adaptor 246 but includes at least one dimension which is different than that of the adaptor 246. For example and as is shown in FIG. 6, the second adaptor 262 includes a thickness T1 which is significantly greater than the thickness T2 of the adaptor 246.

The kit 200 of FIG. 6, may further include an additional fourth member 264 in the form of, for example, a conservative head. The conservative head 264 is utilized for placement on the head of a natural humerus. The conservative head 264 includes a portion of the conservative head 264 having a convex periphery 266 for cooperation with the glenoid fossa 8. The convex periphery 266 may include a first articulating surface 268 defining a generally circular outer periphery and a second articulating surface 270 extending from a portion of the circular outer periphery of the first articulating surface 268.

As shown in FIG. 6, the kit 200 also includes a second convex member 272 for cooperation with stem 102. The kit 200 may also include a second concave glenoid component 274 for cooperation with the natural glenoid 8. The kit 200 may further include a second convex glenoid component 276 as well as the first convex glenoid component 118 of the kit 100 of FIGS. 1-5.

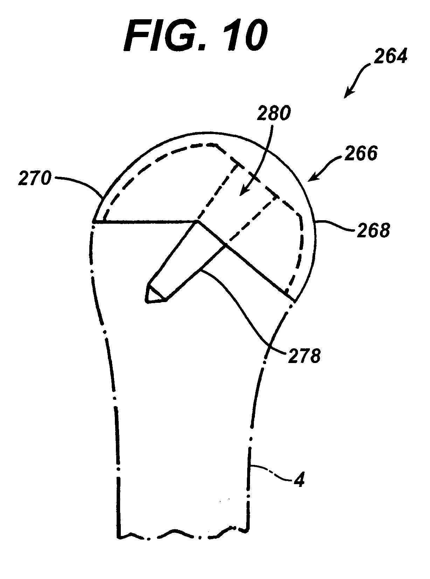

Referring now to FIG. 10, the conservative head 264 is shown in greater detail. The conservative head 264 includes a humeral connecting feature 278 cooperates opposed to the convex surface 266. The humeral connecting feature 278 with a connecting feature 280 formed on the natural humerus 4.

The conservative head 264 may be made of any suitable, durable material and may, for example, be made of a metal. If made of a metal, the conservative a conservative head 264 can be made of a cobalt chromium alloy, a stainless steel alloy, or a titanium alloy.

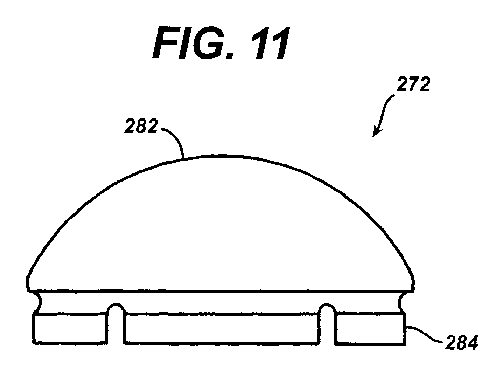

Referring now to FIG. 11, the second convex member 272 is shown in greater detail. The second convex member 272 includes an arcuate periphery 282 which may, as shown in FIG. 11, be in the form of a sector of a sphere and includes a connecting feature 284 extending from the arcuate periphery 282. The connecting feature 284 preferably is similar to the connecting feature 130 of the second member 114 so that the second convex member 272 may be cooperable with the stem 102.

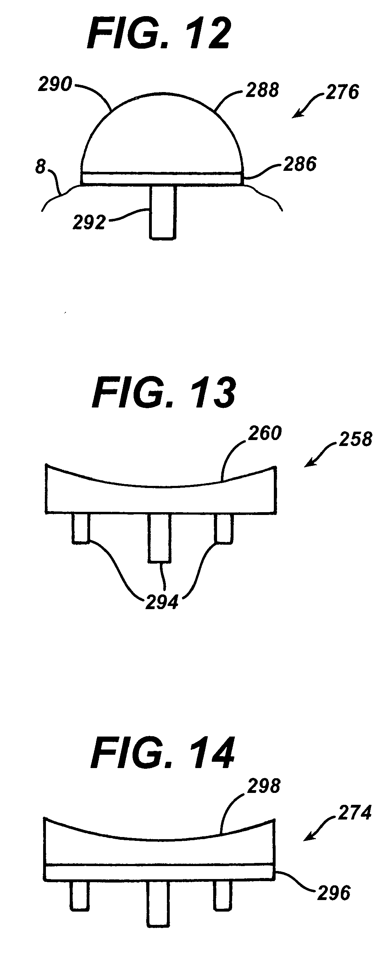

Referring now to FIG. 12, the second convex glenoid component 276 is shown in greater detail. The second convex glenoid component 276 includes a backing member 286 which cooperates with the glenoid fossa 8. The second convex glenoid component 276 further includes a body 288 having an arcuate convex periphery 290. The body 288 may be made of a plastic, for example, a polyethylene, which can be secured to a backing member 286 made of, for example, metal, for example, a cobalt chromium alloy, a stainless alloy, or titanium alloy. The metal backing 286 further include a connecting feature in the form of, for example, post 292.

Referring now to FIG. 13, the first concave glenoid component 258 is shown. The first concave glenoid component 258 may as and is shown in FIG. 13, be of an unitary construction and may be made of, for example, a plastic, for example, an ultra-high molecular weight polyethylene. The concave glenoid component 258 may include a plurality of support features, for example, posts 294 which extend in a direction opposed to the articulating surface 260 of the concave glenoid component 258.

Referring now to FIG. 14, a second concave glenoid component 274 is shown. The second concave glenoid component 274 as is shown in FIG. 14 is construed of a two piece configuration including a backing member 296 to which the bearing component 298 is secured. The backing component 296 may, as is shown in FIG. 14, be made of a metal, for example, a cobalt chromium alloy, a stainless steel alloy, or a titanium alloy. The bearing component 298 may be made of, for example, a plastic such as an ultra-high molecular weight polyethylene.

Referring again to FIG. 6, the kit 200 may further include a second conservative humeral head 299. The second conservative humeral head 299 is different than the first mentioned first conservative head 264 in that the second conservative humeral head 299 is symmetrical and not adapted to treat cuff tear athroplasty.

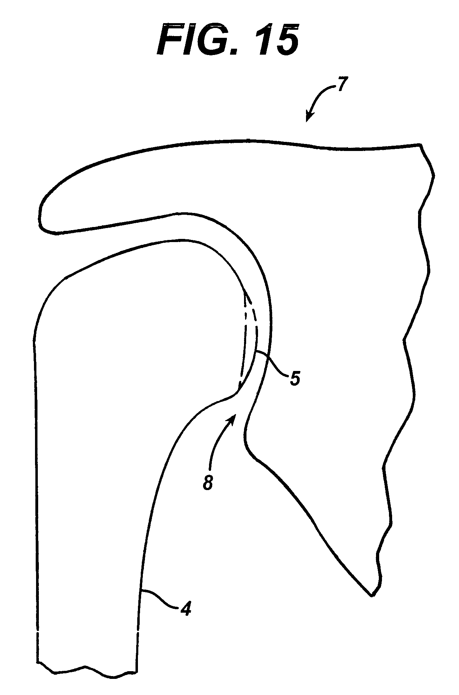

The kit 200 of FIG. 6, may be utilized for shoulder athroplasty for varying disease states of shoulder arthropathy for example different conditions in the progression of osteoarthritis. For example and is shown in FIG. 15-17, the kit 200 of FIG. 6 can be utilized to accommodate three specific disease conditions of osteoarthritis.

The first of these three disease conditions is shown in FIG. 15, where the head 5 of the humerus 4 is worn from a healthy position as shown in solid line to that of a diseased humerus as shown in phantom. The prosthesis that may be chosen for the first condition shown in FIG. 15. The head 5 of the humerus 4 provides for a more anatomical condition. In the shoulder of the condition of FIG. 15, the rotator cuff 7 is in generally good condition.

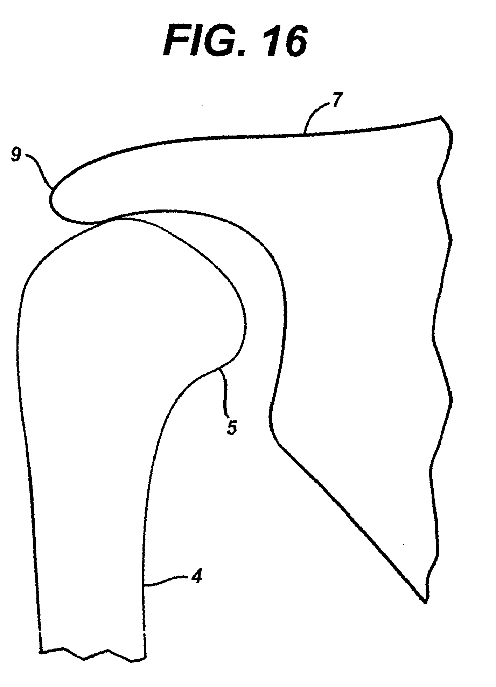

Referring now to FIG. 16, a second disease state of the shoulder is shown with the rotator cuff 7 torn and in which cuff tear arthropathy has occurred such that the head 5 of the humerus 4 has progressed to the point in which the head5 of the humerus 4 articulates against the acromion 9.

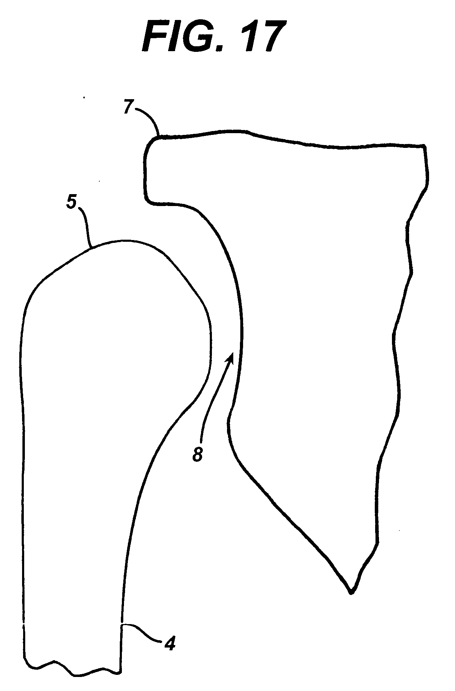

Referring now to FIG. 17, a third disease condition of the shoulder is shown. In this third disease condition the rotator cuff 7 has been severely compromised and the glenoid cavity 8 is grossly mis-shaped. In this third disease state, an alternate design of a shoulder prosthesis is advised.

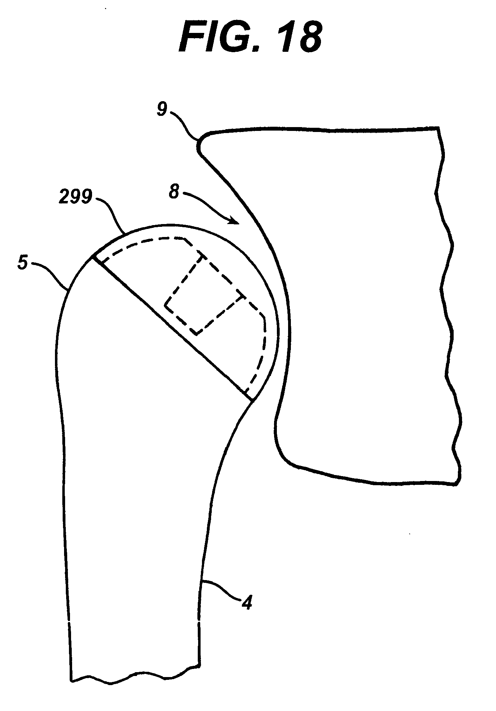

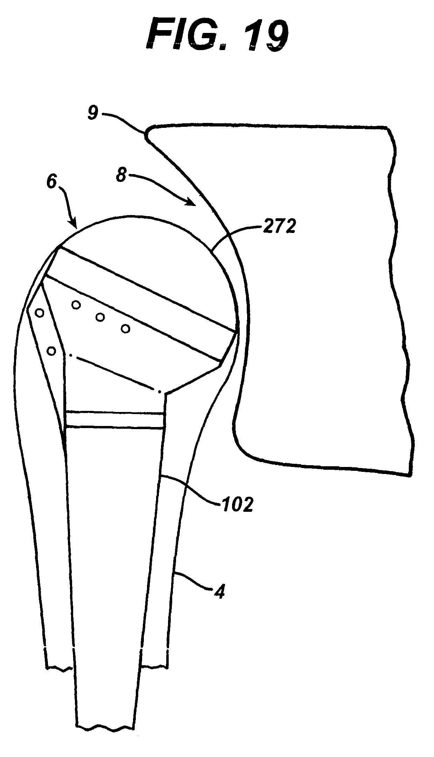

Referring now to FIG. 18 and 19, prostheses is shown for use with the disease state of FIG. 15. Referring now to FIG. 18, the conservative humeral head 299 is shown in position on head 5 of the humerus 4. The use of the conservative humeral head 299 represents a conservative bone sparing procedure.

Referring now to FIG. 19, an alternate prosthesis for use with the first disease condition is shown in FIG. 15. The stem 102 is inserted into the canal 6 of the humerus 4 and the second convex humeral head 272 is secured to the stem 102. The convex humeral head 272 cooperates with the glenoid fossa 8.

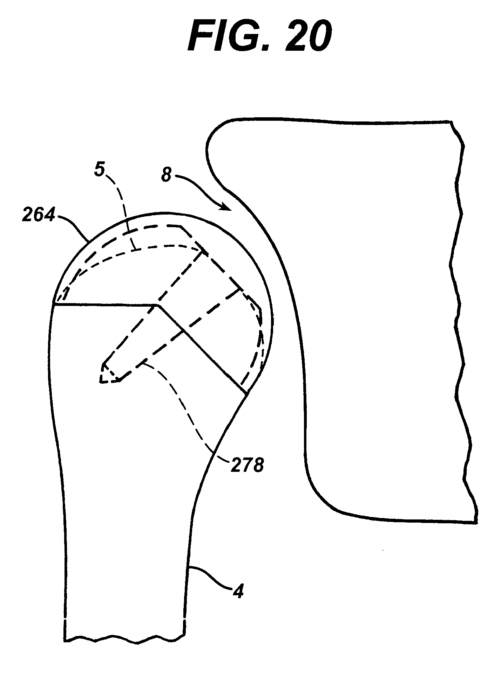

Referring now to FIG. 20-23, alternate embodiments of a prosthesis which is part of the kit 200 of FIG. 6 is shown for use with the second disease condition of FIG. 16.

Referring first to FIG. 20, conservative convex humeral head 264 is shown in position on the humerus 4 for use with the disease condition of FIG. 16. The conservative humeral head 264 presents a conservative or bone sparing approach to the disease condition of FIG. 16. The head 5 of the humerus 4 is prepared to receive conservative humeral 264 and is positioned onto the humeral head 5 of the humerus 4. The conservative humeral head 264 cooperates with, as shown in FIG. 8, the natural glenoid fossa 8.

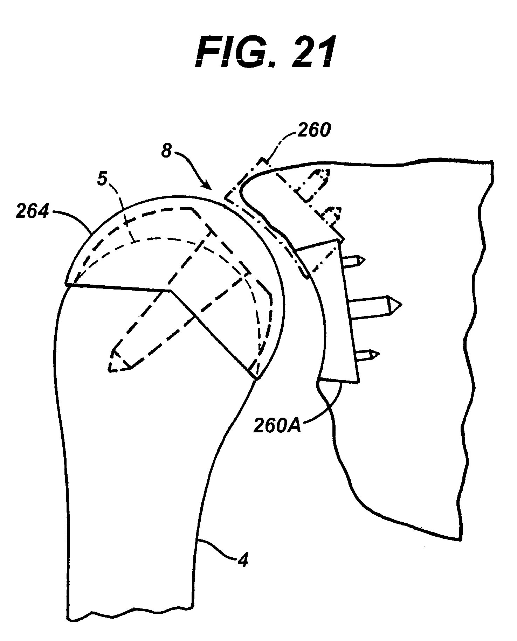

Referring now to FIG. 21, another prosthesis for use in the treatment of the disease condition is shown. As shown in FIG. 21, the conservative humeral head 264 is positioned on head 5 of the humerus 4. The head 264 typically cooperates with the natural glenoid fossa 8. The position of a concave implant 260A (shown in phantom) would not cooperate with the head 264 properly. It should be appreciated that a special glenoid implant 260 may be designed to cooperate with the head 264.

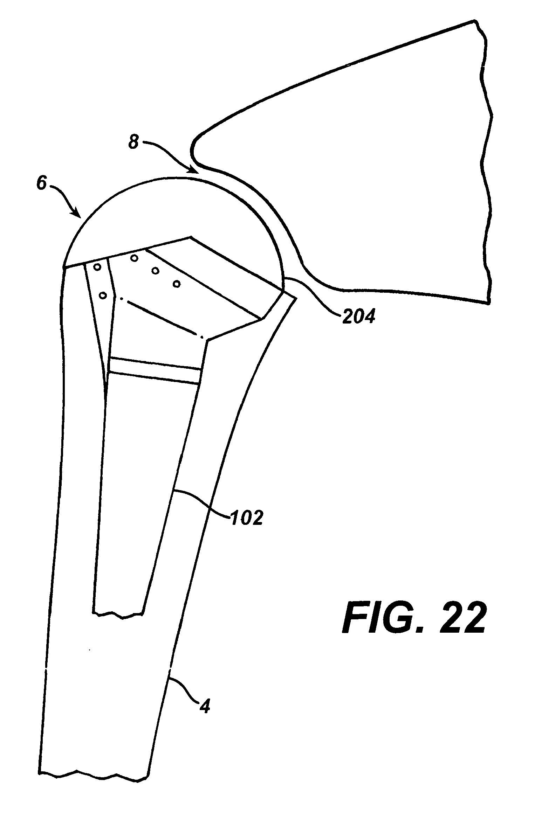

Referring now to FIG. 22, yet another prosthesis for use with the disease condition of FIG. 16 is shown. As is shown in FIG. 22, the stem 102 of the kit 200 of FIG. 6 is positioned in canal 6 of the humerus 4. The head 5 of the humerus 4 is resected to expose the canal 6. An adaptor 246 of the kit 200 of FIG. 6 is positioned on the stem 102. The humeral head 204 of the kit 200 of FIG. 6 is positioned on the adaptor 246. The humeral head 204 cooperates with the natural glenoid 8 as is shown in FIG. 22.

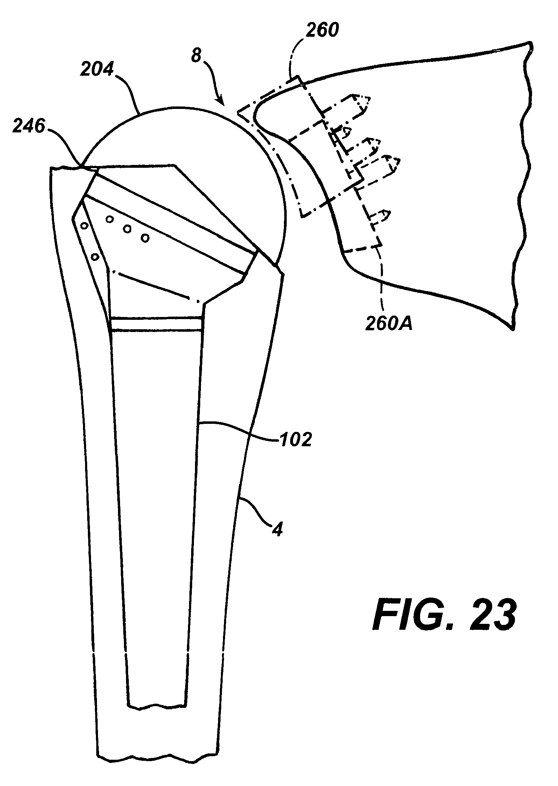

Referring now to FIG. 23, another prosthesis for use disease condition of FIG. 16 is shown. Referring to FIG. 23, the stem 102 of the kit 200 of FIG. 6 is positioned in canal 6 of the humerus 4. The adaptor 246 of the kit 200 of FIG. 6 is positioned on the stem 102 and the humeral head 204 of the kit 200 of FIG. 6 is positioned on the adaptor 246. The head 264 typically cooperates with the natural glenoid fossa 8. The position of a concave implant 260A (shown in phantom) would not cooperate with the head 264 properly. It should be appreciated that a special glenoid implant 260 may be designed to cooperate with the head 264.

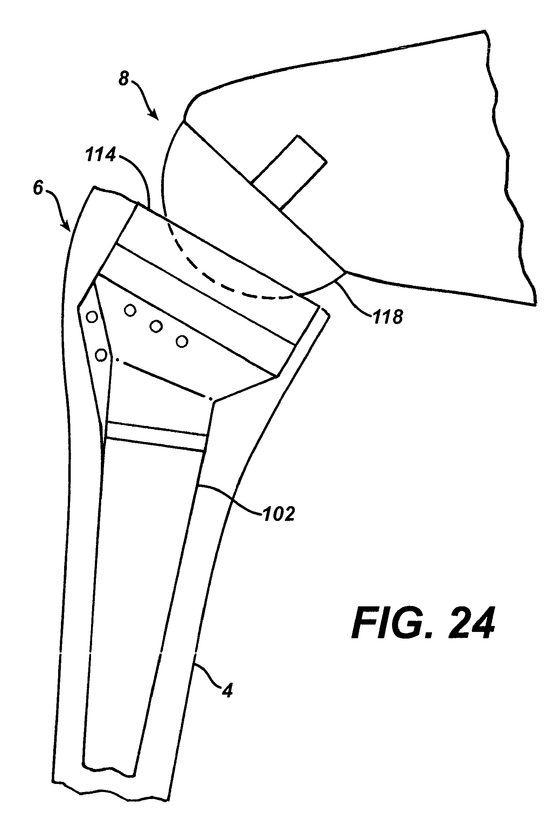

Referring now to FIG. 24, a prosthesis is shown for use with the third disease condition of the FIG. 17. The prosthesis of 24 includes the stem 102 of the kit 200 of FIG. 6, which is positioned in canal 6 of the humerus 4. Concave humeral head 114 of the kit 200 of FIG. 6 is positioned on the stem 102. The convex glenoid component 118 of the kit 200 of FIG. 6 is positioned on glenoid cavity 8. The convex glenoid component 118 articulates with the concave humeral head 114.

Referring now to FIG. 6 as well as FIG. 18-24, it should be appreciated that a wide variety of disease states can be accommodated with the use of the kit 200 of FIG. 6. Further, it should be appreciated that as the condition of a patient deteriorates, a more conservative prosthetic may be removed and replaced with prosthetic implants designed for use with the further progression of the diseased shoulder. For example and referring now to FIG. 18 and 19, the conservative humeral head 299 may be resected with the head 5 of the humerus 4 of FIG. 18 to accommodate the stem 102 and head 272 of FIG. 19. Thus, the prosthesis of FIG. 18 may be replaced with the prosthesis of FIG. 19 on the same patient without requiring the removal of the humeral head 299 from the head 5.

Referring now to FIG. 20-25, the prosthesis of FIG. 20, can be replaced with the prosthesis 21 in the same patient by merely adding the concave glenoid implant 260 to the natural glenoid 8. The conservative humeral head 264 may remain on the humerus 4 of the patient.

Referring now to FIG. 20-23, the conservative humeral head 264 of the prostheses of the FIG. 20 and 21 may be replaced with the prosthesis assembly of FIG. 22 and 23. The head 5 may be resected from the humerus 4 of a patient having the conservative humeral head 264 of FIG. 20-21. The resected humerus 4 may thus receive the humeral stem 102 of FIG. 22 and 23 as well as the adaptor 246 and the humeral head 204. Thus, the prosthesis of FIGS. 20 and 21, may be replaced with the prosthesis of FIGS. 22 and 23 without the trauma of removing the conservative humeral head 264 from the head 5 of the humerus 4.

Referring now to FIG. 22, 23, 24 it should be appreciated that the humeral prosthesis assembly of FIG. 22 and 23 may be replaced with the humeral prosthesis assembly of FIG. 24. For example and as is shown in FIGS. 22, 23, and 24 the humeral stem 102 may remain in the humerus 4 of the patient and the adaptor 246 and the humeral head 204 may be removed from the humeral stem 102 while the humeral stem remains in humerus 4 of the patient. The concave humeral head may be attached to the stem 102 of the humerus 4 as is shown in FIG. 24. The convex glenoid 118 may then be positioned on the glenoid 8 of the patient to provide for the prosthesis of FIG. 24. It should be appreciated that the progression of the shoulder disease from the second disease condition of FIG. 16 to that of the third disease condition 17 may be accommodated without the traumatic removal of the humeral stem 102 of the humerus.

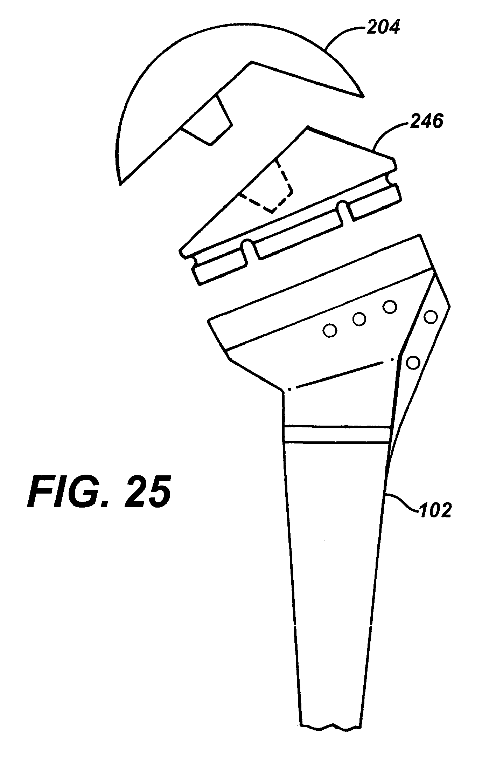

Referring now to FIG. 25, the humeral stem 102, the adaptor 246, and the humeral head 204 are shown in an exploded view.

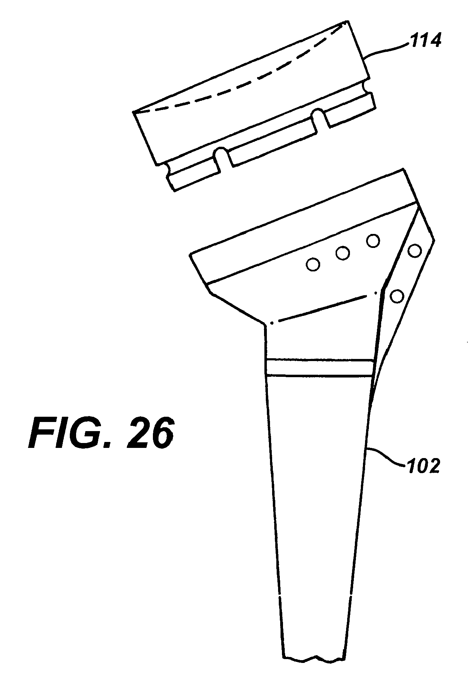

Now referring to FIG. 26, the stem 102 is shown in an exploded view with a concave head 114.

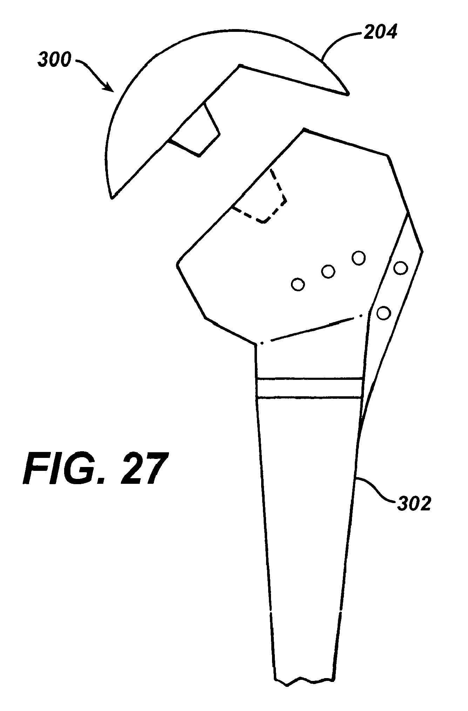

Referring now to FIG. 27, alternate embodiment of an extended articulating convex prostheses is shown as prosthesis 300. The prosthesis 300 includes a stem 302 which is somewhat different than the stem 102 of the kit of FIG. 6. The stem 302 is adapted for receiving the extended articulating first member 204 of the kit 200 of FIG. 6.

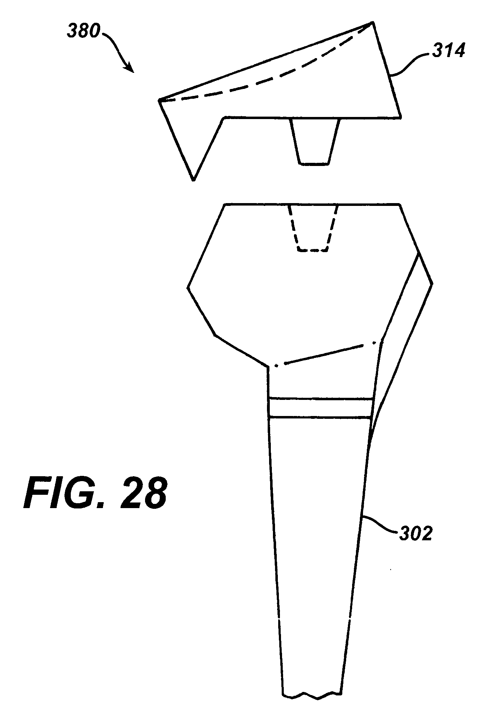

Referring now to FIG. 28, a concave humeral stem assembly 380 is shown. The concave humeral stem assembly 380 of FIG. 28 includes a concave humeral head 314 which is different than the concave humeral head 114 of the kit 200 of FIG. 6. The concave humeral head 314 of FIG. 28 is adapted for use with the stem 302 of FIG. 27.

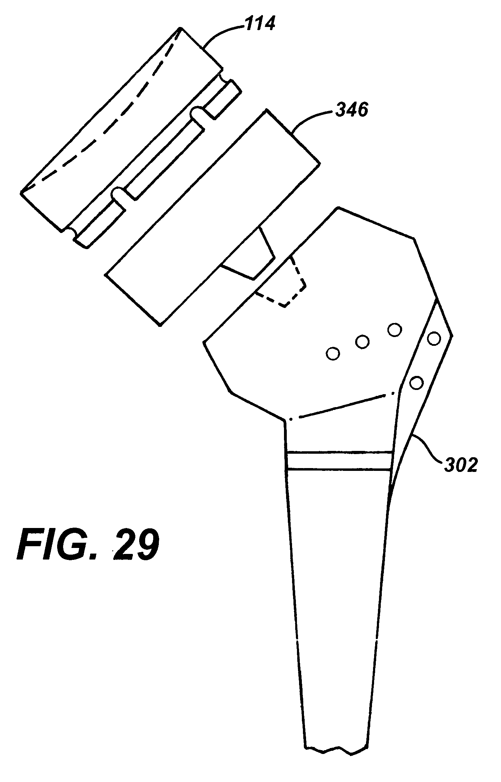

Referring now to FIG. 29, yet another embodiment of the present invention is shown as prosthetic stem assembly 390. The stem assembly 390 includes the stem 302 of FIG. 27 as well as an adaptor 346 for positioning on the stem 302. Concave humeral head 114 of the kit 100 of FIG. 1 may be positioned on the adaptor 346 to form the concave humeral stem assembly 390 of FIG. 29.

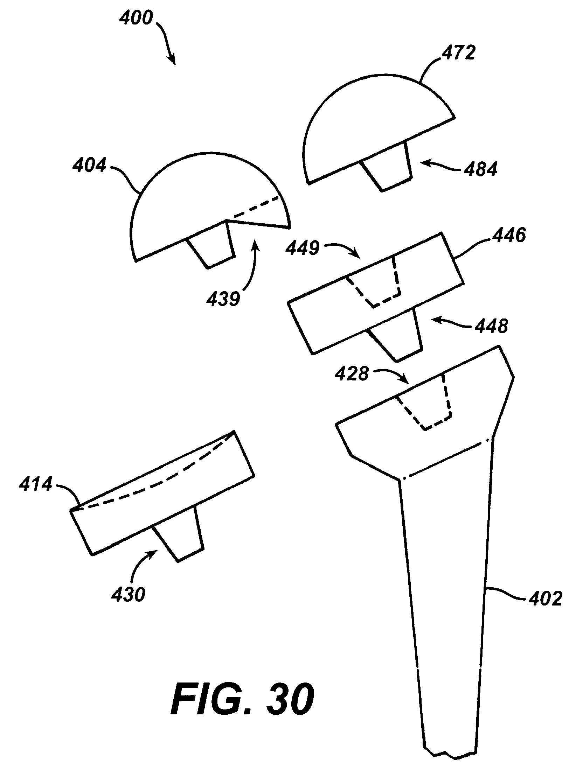

Referring now to FIG. 30, another embodiment of the present invention is shown as kit 400. Kit 400 is similar to kit 200 of FIG. 6 except that kit 400 utilizes different connection mechanisms for various components. For example and is shown in FIG. 30, kit 400 includes a stem 402 similar to the stem 102 of the kit 200 of FIG. 6 except that the stem 402 includes a connector in the form of an internal taper 428. The kit 400 further includes an adaptor 446 similar to the adaptor 246 of the kit 200 of FIG. 6 except that the adaptor 246 includes a first connector 448 in the form of an external protrusion as well as a second connector 449 in the form of a cavity defining an internal taper.

The kit 400 further includes a convex extended articulation head 404 similar to the head 204 of the kit 200 in FIG. 6 except that the extended articulation head 404 includes a protrusion 439 in the form of an external protrusion for cooperation with the tapered cavity 449 of the adaptor 446.

The kit 400 further includes a convex head 472 similar to the head 272 of the kit 200 of FIG. 6 except that the convex head 472 includes a connector in the form of a tapered protrusion 484. The kit 400 further includes a concave head 414 similar to the concave head 114 of the kit 200 of FIG. 6 except that the concave head 414 includes a connector in the form of an external tapered protrusion 430.

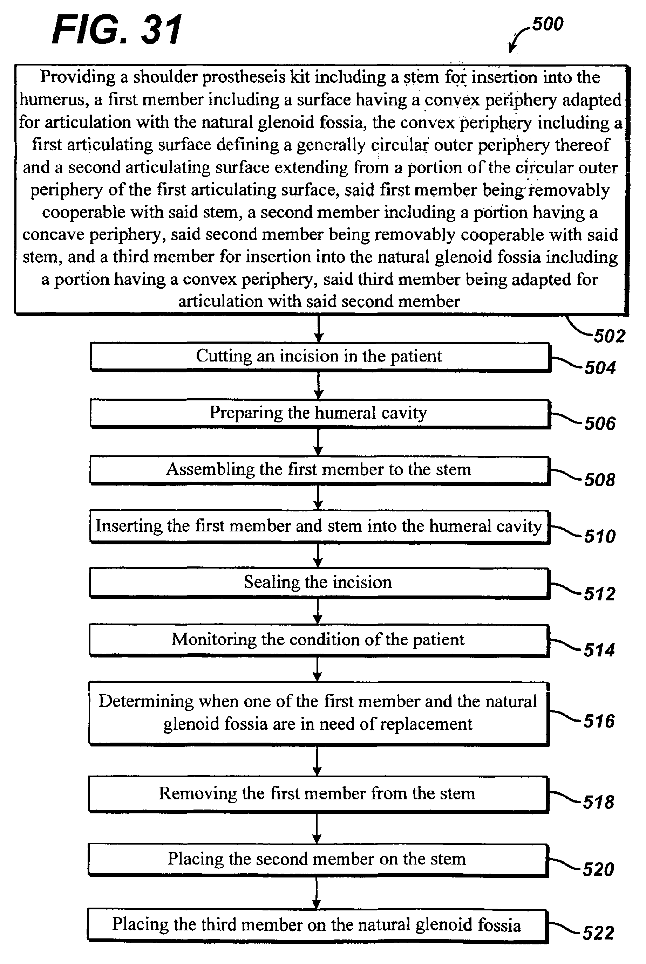

Referring now to FIG. 31, a method 500 for performing shoulder athroplasty is shown. The method 500 includes a first step 502 of providing a shoulder prosthesis kit including a stem for insertion into the humerus. The kit includes a first member having a surface having a convex periphery adapted for articulation with the natural glenoid fossa. The convex periphery includes an articulating surface for defining a generally circular outer periphery and a second articulating surface extending from a portion of the circular outer periphery of the articulating surface. The first member is removably cooperable with the stem.

The kit further includes a second member having a portion with a concave periphery. A second member is removably cooperable with a stem. A kit further includes a third member for insertion into the natural glenoid fossa including a portion having a convex periphery. The third member is adapted for articulating with the second member.

The method 500 further includes a second step 504 of cutting as incision in the patient. The method 500 also includes a third step 506 of preparing the humeral cavity and a fourth step 508 of assembling the first member to the stem. The method 500 further includes a fifth step 510 of inserting the first member and the stem into the humeral cavity.

The method 500 further includes a sixth step 512 of sealing the incision and a seventh step 514 of monitoring the condition of the patient. The method 500 further includes the eighth step 516 of determining when one of the first member and the natural glenoid fossa are in need of replacement. The kit further includes a ninth step 518 of removing the first member from the stem and a tenth step 520 of placing the second member on the stem. The method 500 further includes an eleventh step 522 of placing the third member on the natural glenoid fossa.

## Claims

1. A shoulder arthroplasty kit for providing for shoulder arthroplasty, comprising:
a stem for insertion into the humerus;
a first member including a surface having a convex periphery adapted for articulation with the natural glenoid fossia, the convex periphery including a first articulating surface defining a generally circular outer periphery thereof and a second articulating surface extending from a portion of the circular outer periphery of the first articulating surface, said first member being removably cooperable with said stem;
a second member including a portion having a concave periphery, said second member being removably cooperable with said stem; and
a third member for insertion into the natural glenoid fossia including a portion having a convex periphery, said third member being adapted for articulation with said second member.

2. The shoulder arthroplasty kit of claim 1, further comprising a fourth member for insertion into the natural glenoid fossia including a portion having a concave periphery, said fourth member being adapted for articulation with said first member.

3. The shoulder arthroplasty kit of claim 1, further comprising an adapter postionable between said stem and one of said first member and said second member for cooperation with said stem and said one of said first member and said second member.

4. The shoulder arthroplasty kit of claim 1, wherein the first articulating surface and the second articulating surface are generally in the shape of a sector of a hollow sphere.

5. The shoulder arthroplasty kit of claim 1:
wherein the second articulating surface and the first articulating surface define a boundary portion there between; and
wherein the boundary portion is generally smooth and continuous.

6. The shoulder arthroplasty kit of claim 1:
wherein the second articulating surface defines a second surface periphery opposed to said first body;
wherein the generally circular outer periphery defines a first plane; and
wherein the second surface periphery defines a second plane, the first plane and the second plane being non-coincident.

7. The shoulder arthroplasty kit of claim 1, wherein the first plane and the second plane define an included angle there between.

8. The shoulder arthroplasty kit of claim 7, wherein the included angle is obtuse.

9. The shoulder arthroplasty kit of claim 7, wherein the included angle is about 160 to 118 degrees.

10. The shoulder arthroplasty kit of claim 1, wherein at least one of said stem, said first member and said second member are operably connected to each other by a tapered connection.
